# EUROPEAN PATENT APPLICATION

(11) **EP 4 162 875 A1**
(43) Date of publication of application: **12.04.2023**
(21) Application number: 21201771.9
(22) Date of filing: 08.10.2021
(51) Int. Cl.: A61B 5/15, A61B 5/151, A61B 5/157, B01L 3/00, F16K 99/00

(54) **MICROFLUIDIC LIQUID SAMPLING DEVICE**

(71) Applicant: Homedicus GmbH, 12103 Berlin (DE)
(72) Inventor: Riester, Markus, 65193 Wiesbaden (DE); Diebold, Michael, 14165 Berlin (DE)
(74) Representative: Eisenführ Speiser

(57) **Abstract**

The invention relates to a liquid sampling device (100) comprising a support structure (106) having a liquid reception interface (130) that is in fluid communication with a reservoir chamber (134) and a microfluidic valve (136), the microfluidic valve (136) being connected to a measuring chamber (138). The liquid reception interface (130), the reservoir chamber (134) and the microfluidic valve (136) are configured to allow liquid entering the liquid reception interface to flow into the reservoir chamber (134) while being prevented from passing the microfluidic valve (136) unless a predetermined static pressure difference over the microfluidic valve (136) is exceeded. The microfluidic valve (136) is a passive valve without moving parts. Passing of liquid through the microfluidic valve is controlled by a static pressure difference over the microfluidic valve (136). The reservoir chamber (134) is configured and arranged to cause high enough a static pressure at an entrance side of the microfluidic valve (136) that causes a flow of liquid through the microfluidic valve (136) into the measuring chamber (138) once the reservoir chamber (134) is completely filled.

## Description

The invention refers to a liquid sampling device, in particular a body fluid sampling device, for instance a blood sampling device. The invention further refers to a testing device for testing a body fluid sample.

A body fluid sampling device can take up a liquid body fluid that can be used for testing if at least one predetermined analyte or property of the body fluid exists with the sample.

Blood sampling devices may comprise a skin piercing means, for instance a needle, a hollow needle or a lancet.

A body fluid sampling and testing device may further comprise testing means such as a test chamber with reagents and/or sensors or a lateral flow assay.

In US 8,821,413 B2 a system for withdrawing small amounts of body fluid from an animal or human is disclosed. The system includes a holder and a disposable lancing unit attached to the holder. The lancing unit also includes an open capillary channel for piercing the skin and transporting the body fluid to a detection area.

Cheng Guo Li et al (Lab Chip, 2015, 15, 382-390, DOI: 10.1039/cl4cO0937a) present a self-powered one touch blood extraction system comprising a novel polymer capped hollow microneedle integrated with a pre-vacuum actuator. The entire system is made of low-cost and disposable materials to achieve easy operation with a miniature structure and to meet the challenging requirements for single use application in a point of care system without the use of any external power equipment.

It is an object of the invention to provide an improved liquid sampling device.

According to the invention, this object is achieved with a microfluidic liquid sampling device comprising a support structure having an exterior surface of the device for contacting a liquid sample or a patient. The support structure has a liquid reception interface, e.g. a hole, that is in fluid communication with a reservoir chamber and a microfluidic valve. The microfluidic valve is connected to a measuring chamber. The volume of the measuring chamber corresponds to or equals the volume of liquid needed for testing.

The liquid reception interface, the reservoir chamber and the microfluidic valve are configured to allow liquid entering the liquid reception interface to flow into the reservoir chamber while being prevented from passing the microfluidic valve. The microfluidic valve is passive without moving parts. Passing of liquid through the microfluidic valve is controlled by the static pressure difference (pressure drop, ΔP) over the microfluidic valve. The static pressure difference or pressure drop ΔP needs to exceed a threshold ΔP_{T} to allow entering of body fluid into the measuring chamber.

The reservoir chamber is configured and arranged to cause high enough a static pressure at the entrance side of the microfluidic valve to exceed the threshold static pressure difference ΔP_{T} once the reservoir chamber is completely filled. According, when in use, the liquid, in particular body fluid, e.g. blood, will flow from the fluid reception interface into the reservoir chamber. Once the reservoir chamber is filled, the threshold static pressure difference ΔP_{T} over the microfluidic valve is exceeded and the liquid can flow into the measuring chamber.

Once the measuring chamber is filled, further flow of liquid into the measuring chamber is preferably stopped by injecting an air bubble that separates the liquid in the measuring chamber from the liquid in a liquid passageway between the reservoir chamber and the measuring chamber and/or the microfluidic valve and the measuring chamber.

For injecting the air bubble, an air passageway is provided that preferably branches off the liquid passageway between the measuring chamber and the microfluidic valve.

Buffer solution may be added to the body fluid in the measuring chamber and the body fluid can be tested by means of a lateral flow assay or otherwise.

Preferably, the microfluidic valve has an entrance that is connected to the reservoir chamber by a fluid passageway having cross-sectional dimensions wherein a largest circle fitting in the cross-section has a diameter between 100 µm and 150 µm. Thus, capillary action will cause a fluid transport to the microfluidic valve.

Preferably, the microfluidic valve has an exit that is connected to the measuring chamber by a connecting fluid passageway having cross-sectional dimensions wherein a largest circle fitting in the cross-section has a diameter between 100 µm and 150 µm. Thus, capillary action will cause a fluid transport from the microfluidic valve into the measuring chamber. If the width or diameter of the fluid passageway is sufficiently small, then the combination of surface tension of the liquid (which arises from cohesion within the liquid) and adhesive forces between the liquid and the wall of the fluid passageway act to propel the liquid.

Passageways that have a cross section with dimensions that differ in different directions - for instance a rectangular cross section - it is the diameter of a largest circle fitting in the cross-section that matters. In case the cross section is rectangular, it is the smaller dimension that is relevant for the capillary action.

Preferably, the microfluidic valve has a valve chamber with cross-sectional dimensions wherein a largest circle fitting in the cross-section of the valve chamber has a diameter of more than 200 µm, preferably more than 400 µm. Thus, almost no capillary action will act on the liquid in the valve chamber, thus interrupting fluid transport by way of capillary action. The transition from the small diameter or width of about 80 µm to 160 µm at the entrance of the microfluidic valve to the larger diameter or width of the valve chamber preferably is sharp, i.e. the edge has a radius of less than 10 µm and preferably less than 5 µm.

The liquid sampling device preferably comprises a skin-piercing needle or lancet that preferably is arranged proximate the fluid reception interface. Thus, the liquid sampling device can be pressed on a patients skin for piercing the skin and allowing a drop of blood entering the fluid reception interface.

The measuring chamber preferably has a capacity of 5 µl. This is a useful amount of liquid to be tested for the presence of an analyte.

The reservoir chamber preferably has a capacity of 3 µl.

The support structure with the microfluidic arrangement preferably s an integral part made from thermoplastic material, in particular from polycarbonate, cyclopolyolefins, or poly-(methyl)methacrylate. The material of the fluid passageways and the microfluidic valve in combination with the dimension of the fluid passageways and the microfluidic valve determine the capillary action for a specific liquid to be sampled.

The preferred dimensions and the preferred thermoplastic material are in particular suitable for sampling a liquid similar to water or blood.

These and other aspects of the invention will be illustrated by way of examples with reference to the figures. Of the figures,
- Fig. 1:: shows an exploded view of a testing device that can incorporate the invention;
- Fig. 2:: shows an exploded view of an alternative embodiment of a testing device that can incorporate the invention
- Fig. 3:: shows a cross-sectional view of a testing device illustrating an optical path for inspecting a test result on a lateral flow assay;
- Fig. 4:: shows a partially cut-away model of a testing device that can incorporate the invention;
- Fig. 5:: shows an embodiment of a microfluidic arrangement that can be incorporated in a testing device according to figures 1,2 and 4 for fluid sampling;
- Fig. 6a to d:: illustrates steps occurring during use of the microfluidic arrangement of figure 5;
- Fig. 7:: is cross-sectional view of an alternative embodiment of the microfluidic system;
- Fig. 8:: is a detailed view of the microfluidic valve of the alternative embodiment of the microfluidic system;
- Fig. 9a, b:: is a schematic illustration of the "opening" of the microfluidic valve of the microfluidic arrangement of figures 5 and 6;
- Fig. 10:: illustrates the "open" microfluidic valve schematically; and
- Fig. 11a, b:: illustrates the cross-section of different parts of the microfluidic system and shows the largest circle that fits in the respective cross section.

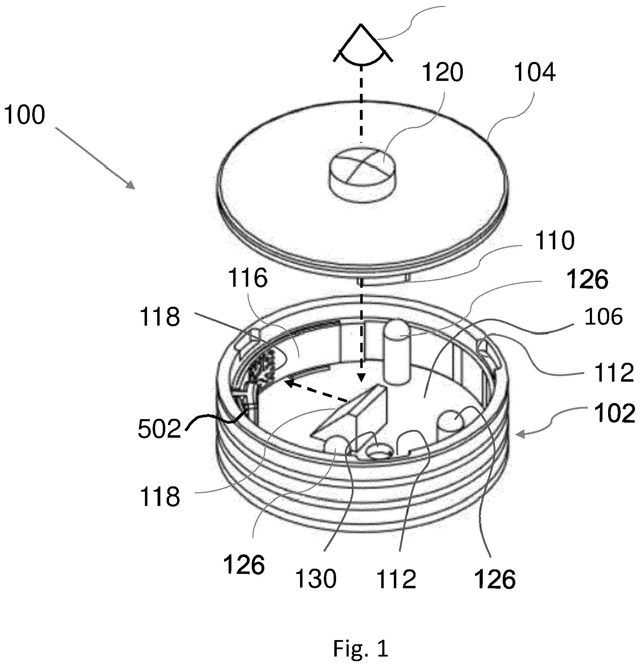

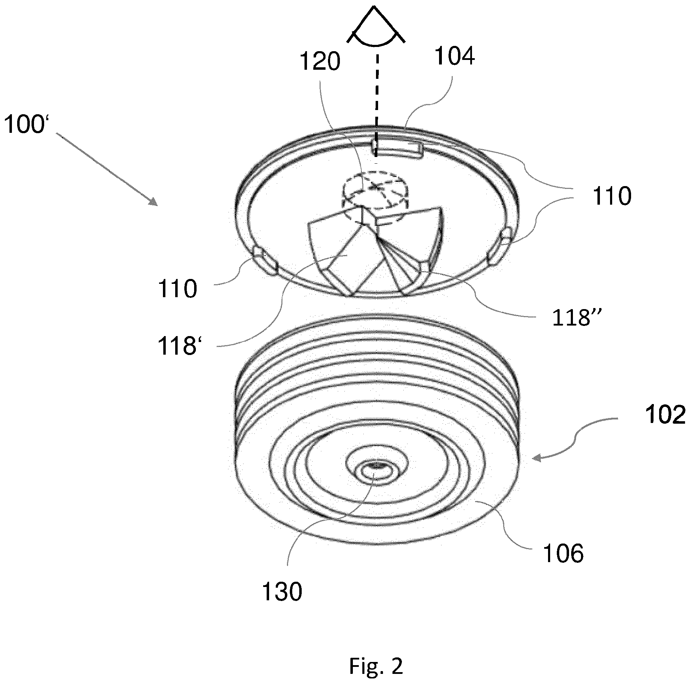

A testing device 100 as shown in figure 1 is comprised of a testing assembly 102 and a cover unit 104. The testing assembly 102 comprises a support structure 106 and a wall 108. The cover unit 104 can be attached to the testing assembly 102 for instance by means of protrusions 110 can that are arranged along the periphery of the cover unit 104 and corresponding recesses 112 in the wall 108. The testing assembly is configured to hold a testing strip 114, in particular a testing strip 114 for a lateral flow assay. The testing strip 114 comprises a test portion 116. A reflector 118 allows observing the test portion 116 via an observation window 120 in centre of the cover unit 104. The observation window 120 may comprise a lens that has a focal lens corresponding to the length of the optical path from the lens 120 via reflector 118 to the test portion 116. The reflector 118 can be arranged on the support structure 106 as in figure 1. Alternatively or in addition, one or more reflectors 118' or 118" can be arranged only inside of cover unit 104 as shown in figure 2.

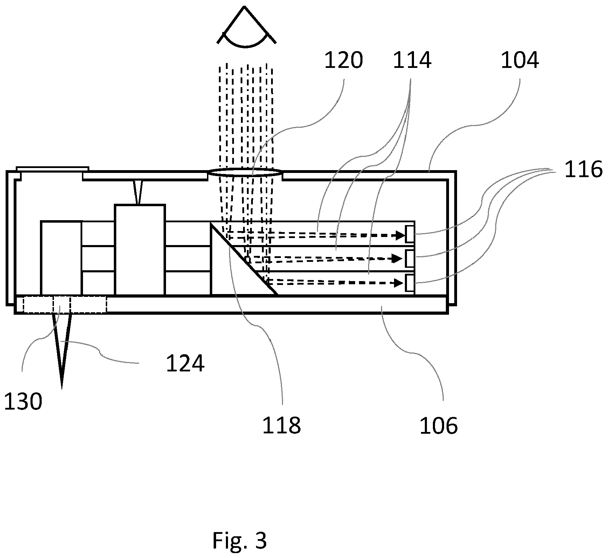

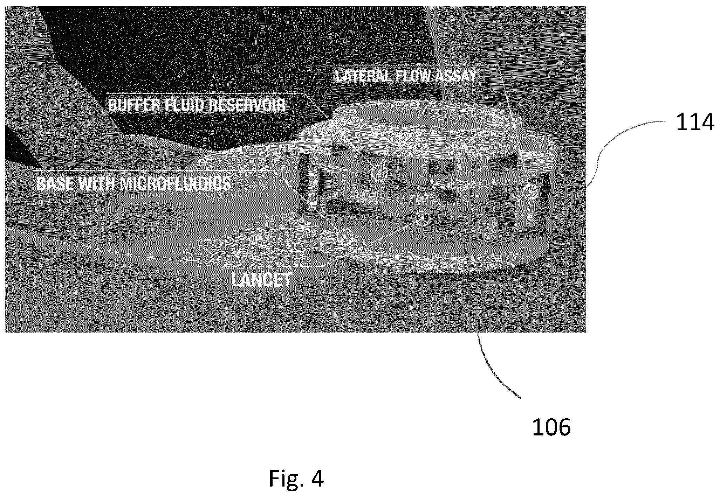

The optical inspection of one or more testing strips 114 of testing device 100 is illustrated in figure 3. As can be taken from figure 4, the test portion 116 of testing strip 114 can be observed via lens 120 and the reflector 118.

Figure 4 illustrates an example of a testing device in a partial cut away representation.

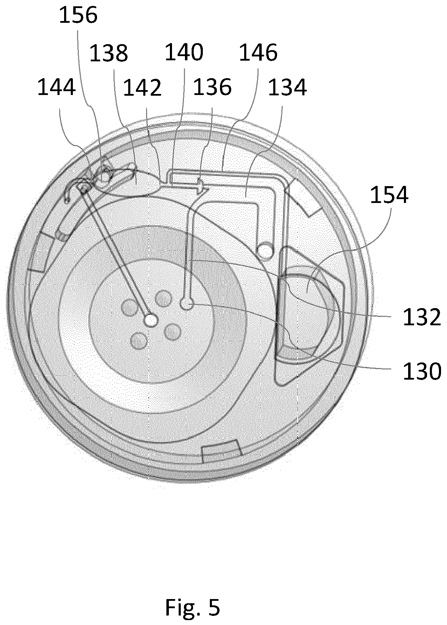

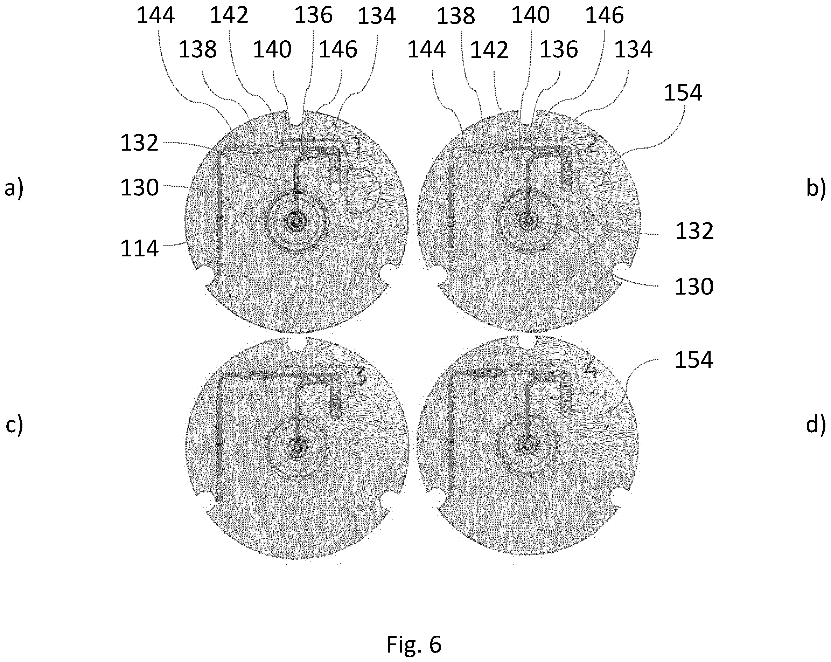

Figure 3 further illustrates an optional needle 124 that can be used for piercing a patient's skin to thus extract a drop of blood. Instead of the needle 124, a lancet or a hollow needle can be provided. The needle 124 is arranged next to a liquid sample reception interface 130 that may be implemented as a hole in the support structure 106. The hole in the support structure 106 is fluidly connected to a microfluidic arrangement that is integrated in the support structure 106 and will be further illustrated by way of example as shown in figures 5 and 6.

The testing device 100 is configured to take up a liquid sample and to convey the liquid sample to a testing unit that may comprise the testing strip 114 or other testing means for instance electronic testing means comprising sensors or the like.

For absorbing a liquid sample, for instance a body fluid such as blood, the liquid reception interface 130 is arranged in the support structure 106. The liquid reception interface 130 can be a hole that is connected to a fluid passageway 132, which is in fluid communication with a reservoir chamber 134 and a microfluidic valve 136. The microfluidic valve 136 is arranged between the reservoir chamber and a measuring chamber 138. The microfluidic valve 116 is fluidly connected to the measuring chamber 138 by a short connection fluid passageway 140 that extends between the microfluidic valve 136 and the fluid entrance 142 of fluid chamber 138. Fluid chamber 138 has a fluid exit 144 that is fluidly connected with the test unit, for instance a testing strip 114. An air passageway 146 branches off the short connection fluid passageway 140. By means of the air passageway 146, an air bubble can be injected into the short fluid passageway 140 to thus separate liquid in the measuring chamber 138 from any liquid in the microfluidic valve 136 or the reservoir chamber 134.

In the testing assembly buffer fluid reservoirs 126 are arranged so that a liquid sample to be tested can be mix with buffer fluid flowing of the liquid sample can be facilitated by means of buffer fluid.

As shown in figure 5 and 6 the liquid sample reception interface 130, on the fluid passageway 132, the reservoir chamber 134, the microfluidic valve 136, the short fluid passageway 140, the measuring chamber 136 and the air passageway 146 are all integrated in the support structure 106.

Next to the liquid sample reception interface 130 a piercing means such as needle 124 or a lancet may be arranged. Such piercing means may be used to pierce a patient's skin to thus extract a drop of blood. The needle can be a hollow needle that is connected to the fluid passageway 132 via the liquid sample reception interface 130.

For testing a liquid sample, the liquid sample is brought in fluid contact with the liquid sample reception interface 130. Capillary forces cause the liquid sample to flow into the reservoir chamber 136. Accordingly, a body fluid can pass via the liquid reception interface 130 and fluid passageway 132 into the reservoir chamber 134; see figure 6a.

While flowing into reservoir chamber 134, the liquid sample is in contact with the microfluidic valve 136 but cannot pass the microfluidic valve 136 because initially, the pressure drop of static pressure over the microfluidic valve 136 is too low.

Only when the reservoir chamber 134 is completely filled, the static pressure on the entrance side of the microfluidic valve 136 is high enough to cause enough of a pressure drop over the microfluidic valve 136 for exceeding the threshold pressure drop. Then, liquid can pass the microfluidic valve 136 and flow via the short fluid passageway 140 into the measuring chamber 138; see figures 6b and 6c.

The purpose of the reservoir chamber 134 and the microfluidic valve 136 is to ensure that an initial portion of the liquid sample is prevented from entering the measuring chamber 138 but received in the reservoir chamber 134. This is, because an initial portion of some liquid sample, for instance an initial portion of extracted blood is less suitable for an analysis or for testing then a further portion of the same liquid sample.

Once the measuring chamber 138 is completely filled (figure 6c), further flowing of liquid sample into the measuring chamber can be stopped by injecting an air bubble via the air passageway 146 into the short fluid passageway 140; see figure 6d. The air bubble in the short fluid passageway 140 separates the liquid sample in the measuring chamber 138 from further liquid at the microfluidic valve 136 or in the reservoir chamber 134.

Once the measuring chamber 138 is completely filled and an air bubble separates a liquid sample in the measuring chamber 138 from further liquid (figure 6d), the liquid sample in the measuring chamber can be mixed with a buffer solution and fed to the test unit, for instance to the testing strip 114.

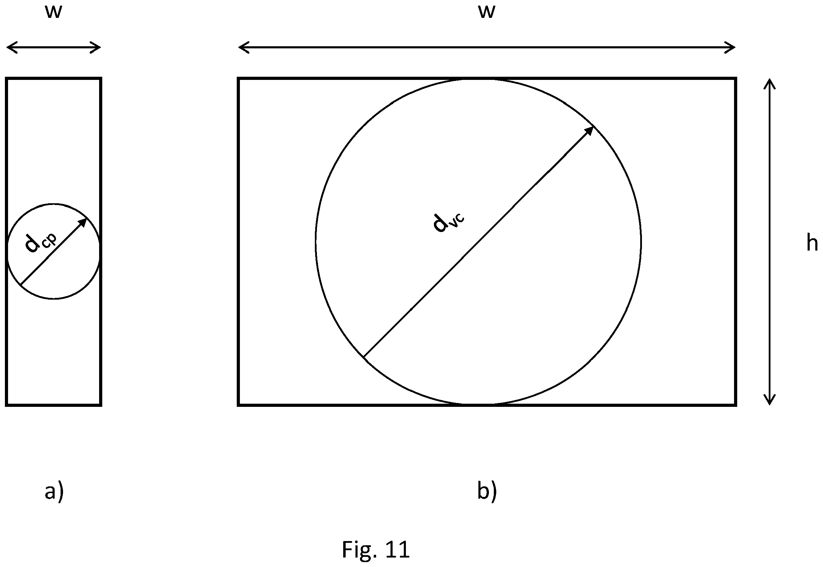

As illustrated in figures 11a and 11b, all passageways and chambers of the microfluidic system have rectangular dimensions with an identical height h. Figures 5 to 10 show plan views of embodiments of the microfluidic systems and its. In these plan views, the varying width w of the passageways and chambers is visible. Since all passageways and chambers have the same height h, it is their width w that determines the capillary action.

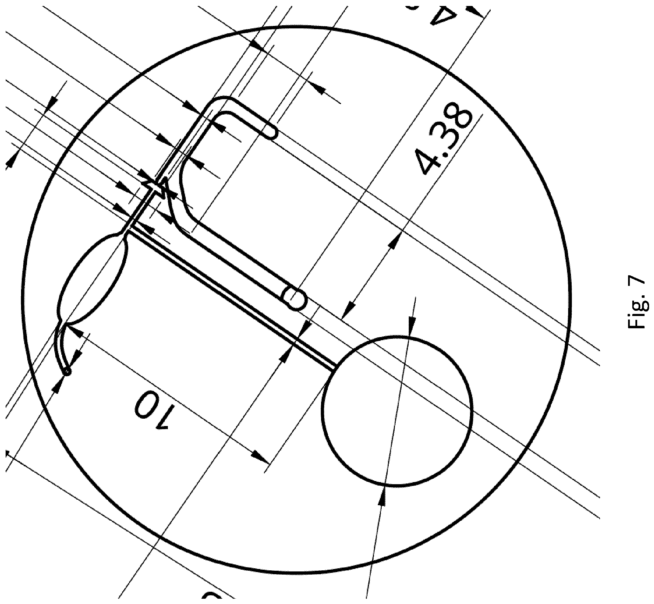

The operation of the microfluidic assembly depends on capillary action and thus on the cross-sectional dimensions of the fluid passageways and of the microfluidic valve, the surface tension of the liquid to be sampled and the contact angle between the liquid-vapour interface and the solid inner surface of the fluid passageway and the microfluidic valve surface. The narrower the fluid passageway the larger is the capillary action. Hence, the liquid to be sampled will be transported to an entrance 148 of the microfluidic valve 136 (see figure 7a) but cannot overcome the microfluidic valve due to an increase in width at the entrance to a valve chamber 150 of the microfluidic valve; see figure 7b. The transition from the small width of about 80 µm to 160 µm at the entrance 148 of the microfluidic valve to the larger width of the valve chamber 150 preferably is sharp, i.e. the edge (see detail A in figure 7b) preferably has a radius of less than 10 µm or even more preferred less than 5 µm. The increased width results in a reduced capillary action and liquid will only flow into the valve chamber 150 of the microfluidic valve 136 when a certain level of hydrostatic pressure exists in the liquid at the entrance 148 of the valve chamber 150. Therefore, a raise of the static pressure on the liquid at the entrance 148 of the microfluidic valve 136 is needed for the liquid to flow into the valve chamber, thus passing the microfluidic valve. Such increased hydrostatic pressure is reached if the reservoir chamber 134 is completely filled.

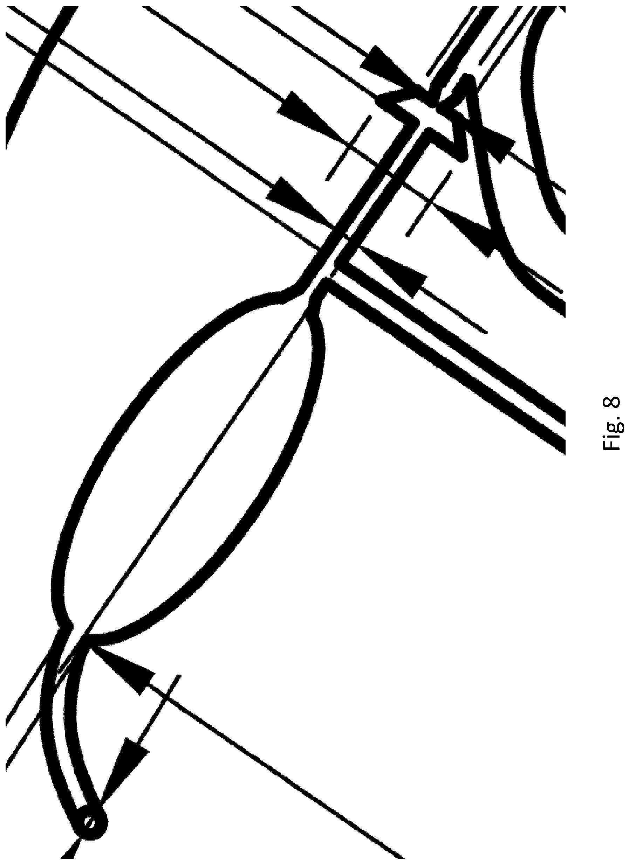

Once the valve chamber 150 is filled with liquid (see figure 8), further transport of the liquid is again accomplished by capillary action in the connecting fluid passageway 140. Accordingly, the microfluidic valve will not "close" again on its own.

The microfluidic arrangement in the support structure 106 thus provides for reliable and reproducible sampling for instance of a body fluid such as blood.

The microfluidic arrangement in the support structure 106 preferably is made from thermoplastic material, in particular polycarbonate, cyclopolyolefins, poly-(methyl)methacrylate.

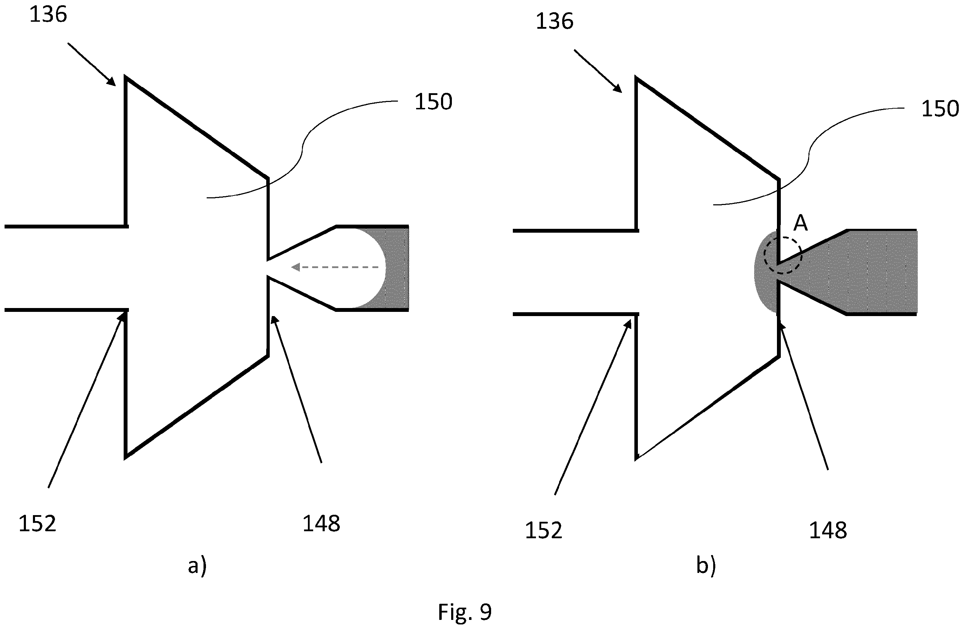

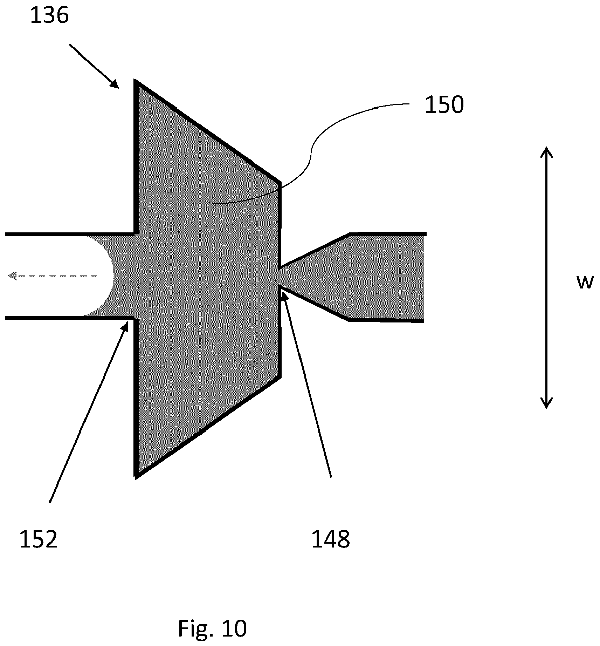

Figures 9 and 10 illustrate a cross-sectional views of the microfluidic valve 136. The valve chamber 150 that has a larger width than a passageway at the entrance 148 of the microfluidic valve 136. The connecting passageway 140 at the exit 152 of the microfluidic valve 136 again has a small width of about 120 µm. The dimensions of the microfluidic valve preferably are as follows:

| | |
|---|---|
| width at the entrance: | 120 µm |
| length of the valve chamber: | >100 µm, for instance 200 µm |
| maximum width of the valve chamber: | > 200 µm |
| width at the exit: | 120 µm |

Figure 11 illustrates the cross section of the microfluidic valve and the passageways. In the embodiments of figures 5, 6, 7 and 8 all chambers, passageways and the microfluidic valve have rectangular cross sections with the same height *h* of 200 µm. In alternative embodiment, the height *h* of the chambers, passageways and the microfluidic valve is between 180 µm and 320 µm. The capillary action occurs depending on the width *w* of the cross section. If the opposite walls of a passageway are close enough, capillary action occurs. Since in a rectangle, two opposing walls are closer to each other than the other two opposing walls, the distance between those walls that are closer to each other matters. The capillary action may occur if a largest circle that fits in the cross section of a passageway is small enough, see figure 11. In figure 11, the passageway is small enough to cause capillary action. The largest circle fitting in the cross section has a small diameter *d*. In figure 11b, no capillary action occurs because of too large a distance between the closest opposing walls. The diameter d of largest circle fitting in the cross section is larger than in figure 11a.

The volume of the reservoir chamber preferably is at least 3 mm³ (i.e.3 µl) and preferably at most 5 mm³ (i.e. 5 µl).

The volume of the measuring chamber preferably is 1 mm³ (i.e. 1 µl).

The fluid passageway 132 between the fluid reception interface 130 and the reservoir chamber 134 have a width between 250 µm and 350 µm.

For injecting the air bubble, an air chamber 154 is provided that can be manually compressed by a user. The air chamber 154 is connected to air passageway 146.

To enable a user to see when the measuring chamber is completely filled and thus injecting the air bubble should be manually triggered, an inspection hole 156 is provided close to the exit 146 of the measuring chamber 138. Once the measuring chamber 138 is completely filled, the liquidity to be sampled, for instance blood, can be seen by a user through the hole 156. Then, the user can actuate the pressure chamber 154 to inject an air bubble into the connecting passageway 140.

The testing device 100 preferably comprises actuating means for the needle or lancet 124. The actuating means preferably provide for a snapping action and an automatic retraction of the needle or lancet 124. In particular, actuating means a disclosed in European patent application EP 20204788 titled "Hand operated actuator mechanism".

### Reference numerals

- 100: testing device
- 102: testing assembly
- 104: cover unit
- 106: support structure
- 108: wall
- 110: protrusion
- 112: recess
- 114: testing strip
- 116: test portion
- 118, 118', 118": reflector
- 120: observation window, lens
- 124: needle
- 126: buffer fluid reservoirs
- 130: liquid reception interface
- 132: fluid passageway
- 134: reservoir chamber
- 136: microfluidic valve
- 138: measuring chamber
- 140: connecting fluid passageway
- 142: fluid entrance
- 144: fluid exit
- 146: air passageway
- 148: valve entrance
- 150: valve chamber
- 152: valve exit
- 154: air chamber
- 156: inspection hole

## Claims

1. Liquid sampling device (100) comprising a support structure (106) with a microfluidic arrangement having a liquid reception interface (130) that is in fluid communication with a reservoir chamber (134) and a microfluidic valve (136), the microfluidic valve (136) being connected to a measuring chamber (138),
wherein
the liquid reception interface (130), the reservoir chamber (134) and the microfluidic valve (136) are configured to allow liquid entering the liquid reception interface to flow into the reservoir chamber (134) while being prevented from passing the microfluidic valve (136) unless a predetermined static pressure difference over the microfluidic valve (136) is exceeded,
the microfluidic valve (136) is a passive valve without moving parts, passing of liquid through the microfluidic valve is controlled by capillary action and a static pressure difference over the microfluidic valve (136), and
the reservoir chamber (134) is configured and arranged to cause high enough a static pressure at an entrance side of the microfluidic valve (136) that causes a flow of liquid through the microfluidic valve (136) into the measuring chamber (138) once the reservoir chamber (134) is completely filled.

2. Liquid sampling device according to claim 1, wherein an air passageway (146) branches off a connection fluid passageway (140) connecting the microfluidic valve (136) and the measuring chamber (138), the air passageway (136) connecting to injection means (154) for injecting an air bubble via the air passageway (146) into the connection fluid passageway (140).

3. Liquid sampling device according to claim 1 or 2, wherein a measuring chamber exit (146) is connected to a test unit.

4. Liquid sampling device according to claim 3, wherein the test unit comprises a liquid flow assay (114).

5. Liquid sampling device according to at least one of claims 1 to 4, wherein a microfluidic valve entrance (148) is connected to the reservoir chamber (134) by a fluid passageway having a width between 100 µm and 150 µm.

6. Liquid sampling device according to at least one of claims 1 to 5, wherein a microfluidic valve exit (152) is connected to the measuring chamber (138) by a connecting fluid passageway (140) having a width between 100 µm and 150 µm.

7. Liquid sampling device according to at least one of claims 1 to 6, wherein a microfluidic valve has a valve chamber (150) having a width of more than 200 µm, preferably more than 400 µm.

8. Liquid sampling device according to at least one of claims 1 to 6, wherein a transition from the small width of about 80 µm to 160 µm at the entrance (148) of the microfluidic valve (136) to the larger width of the valve chamber (150) is sharp, i.e. the edge at the transition has a radius of less than 5 µm.

9. Liquid sampling device according to at least one of claims 1 to 8, further comprising a skin-piercing needle or lancet (124).

10. Liquid sampling device according to claim 9, wherein the needle or lancet (124) is arranged proximate the fluid reception interface (130).

11. Liquid sampling device according to at least one of claims 1 to 10, wherein the measuring chamber (138) has a capacity of between 0,8 to 1,2 µl, in particular 1µl.

12. Liquid sampling device according to at least one of claims 1 to 11, wherein the reservoir chamber (134) has a capacity of between 2,5 and 3 µl, in particular 3 µl.

13. Liquid sampling device according to at least one of claims 1 to 12, wherein the support structure (106) with the microfluidic arrangement is an integral part made from thermoplastic material, in particular from polycarbonate, cyclopolyolefins, poly-(methyl)methacrylate.
